(19) Europäisches Patentamt
European Patent Office
Office européen des brevets

(11) Veröffentlichungsnummer: **0 279 283**
**A2**

(12) **EUROPÄISCHE PATENTANMELDUNG**

(21) Anmeldenummer: **88101572.1**

(51) Int. Cl.⁴: **C07D 237/04**, A61K 31/50

(22) Anmeldetag: **04.02.88**

(30) Priorität: **17.02.87 DE 3704879**

(43) Veröffentlichungstag der Anmeldung:
**24.08.88 Patentblatt 88/34**

(84) Benannte Vertragsstaaten:
**CH DE ES FR GB IT LI NL**

(71) Anmelder: **Merck Patent Gesellschaft mit beschränkter Haftung**
**Frankfurter Strasse 250**
**D-6100 Darmstadt(DE)**

(72) Erfinder: **Prücher, Helmut**
**Königsbergerstrasse 9**
**D-6148 Heppenheim(DE)**

(54) **Pyridazinonderivate.**

(57) Pyridazinonderivate der Formel I

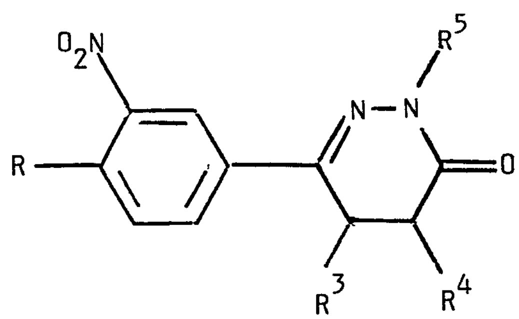

I

worin

R F, Cl, Br, J oder $R^1R^2N$,

$R^1$ und $R^2$ jeweils H, Alkyl oder Benzyl,

$R^3$, $R^4$ und $R^5$ jeweils H oder Alkyl bedeuten und

die Alkylgruppen Jeweils 1-4 C-Atome enthalten, worin jedoch nur dann gleichzeitig R = Benzylamino, $R^3$ = $CH_3$ und $R^4$ = H sein können, wenn $R^5$ Alkyl bedeutet,

sowie deren Salze zeigen positiv-inotrope und vasodilatierende Eigenschaften und können insbesondere als Zwischenprodukte verwendet werden.

EP 0 279 283 A2

# Pyridazinonderivate

Gegenstand der Erfindung sind neue Pyridazinonderivate der Formel I

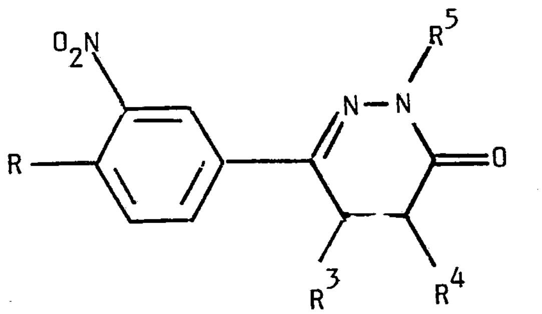

I

worin

R F, Cl, Br, J oder $R^1R^2N$,

$R^1$ und $R^2$ jeweils H, Alkyl oder Benzyl,

$R^3$, $R^4$ und $R^5$ jeweils H oder Alkyl bedeuten und

die Alkylgruppen jeweils 1-4 C-Atome enthalten, worin jedoch nur dann gleichzeitig R = Benzylamino, $R^3$ = $CH_3$ und $R^4$ = H sein können, wenn $R^5$ Alkyl bedeutet,

sowie deren Salze.

Eine ähnliche Verbindung "5-Methyl-6-(3-nitro-4-benzylamino-phenyl)-4,5-dihydro-2H-pyridazin-3-on" (entsprechend I, R = Benzylamino, $R^3$ = $CH_3$, $R^4$ = $R^5$ = H) ist in der DE-OS 2922336 erwähnt, ohne daß dort jedoch ein Verfahren zu ihrer Herstellung angegeben ist.

Der Erfindung lag die Aufgabe zugrunde, neue Verbingungen mit wertvollen Eigenschaften aufzufinden, insbesondere solche, die zur Herstellung von Arzneimitteln verwendet werden können.

Es wurde gefunden, daß die Verbindungen der Formel I und ihre Salze bei guter Verträglichkeit wertvolle pharmakologische Eigenschaften besitzen. Insbesondere zeigen sie eine Wirkung auf die Herzkraft (positiv inotrope Wirksamkeit); ferner wirken die Substanzen vasodilatierend und fördern daher die Durchblutung. Die vasodilatierende und die Herzwirkung kann z.B. an narkotisierten oder wachen Hunden, Katzen, Affen oder Minischweinen, die positiv inotrope Wirkung auch an isolierten Herzpräparationen (z.B. Vorhof, Papillarmuskel oder perfundiertes Ganzherz) von Ratte, Meerschweinchen, Katze oder Hund ermittelt werden, z.B. nach Methoden, wie sie in Azneimittelforschung, Band 31 (I) Nr. 1a (1981), Seiten 141 bis 170, oder von Schliep et al. im 9th International Congress of Pharmacol., London, Abstracts of papers 9P, beschrieben sind.

Weiterhin treten antithrombotische, thrombozytenaggregationshemmende und die Erythrozytenform beeinflussende Eigenschaften auf. Die Beeinflussung der Thrombozytenfunktion im Sinne einer Aggregationshemmung kann an der Ratte ex vivo im Test nach Born (Nature 194, 927-929, 1962) nachgewiesen werden. Die antithrombotische Wirkung zeigt sich in der Verlängerung der Blutungszeit nach Stella (Thrombos. Res. 7, 709-716, 1975), in der Verminderung des Thrombusgewichtes bei der kälteinduzierten Thrombosierung der Jugular-Vene bei der Ratte nach Meng (Ther. Ber. 47, 69-79, 1975) und der Erhöhung der zur vollständigen Thrombosierung notwendigen Laserimpulse an der Mesenterial-Venole der Ratte, entsprechend einer Abwandlung der Methode nach Kovacs (Microvasc. Res. 6, 194-201, 1973).

Die günstige Wirkung auf die Erythrozytenverformbarkeit ist im Nucleoporefilter nach Schmid-Schönbein (Pflüger's Archiv 338, 93-114, 1973) nachweisbar. Auch lassen sich günstige Effekte auf die Fibrinolyse/Euglobulinlysiszeit nach V. Kaulla (Progr. Chem. Fibrinol. Thrombol. 1, 131-149, 1975; ed. J.F. Davidson, Raven Press, N.Y.) feststellen.

Die Verbindungen können dahe als Arzneimittelwirkstoffe in der Human-und Veterinärmedizin verwendet werden. Insbesondere können sie aber als Zwischenprodukte zur Herstellung weiterer Arzneimittelwirkstoffe verwendet werden, z.B. derjenigen, die in der DE-OS 35 05 609 beschrieben sind. So kann man z.B.

durch Hydrierung von Verbindungen der Formel I, worin R eine $R^1R^2N$-Gruppe bedeutet, die entsprechenden 3-Aminoderivate erhalten, z.B. 6-(3,4-Diaminophenyl)-5-methyl-2,3,4,5-tetrahydropyridazin-3-on [vgl. DE-OS 35 05 609, Beispiel 1; dort als "5-Methyl-6-(3,4-diaminophenyl)-4,5-dihydro-pyridazin-3-on" bezeichnet].

In den Formeln ist Alkyl vorzugsweise unverzweigt, hat vorzugsweise 1-3 C-Atome und steht bevorzugt für Methyl, ferner bevorzugt für Ethyl oder Propyl, weiterhin für Isopropyl, Butyl, Isobutyl, sek.-Butyl oder tert.-Butyl.

Die Gruppe $R^1R^2N$ ist vorzugsweise Amino, Methylamino, Ethylamino, Benzylamino, N-Benzyl-N-methylamino oder N-Benzyl-N-ethylamino, ferner bevorzugt Propylamino, Dimethylamino, Methylethylamino, Diethylamino oder Dipropylamino, weiterhin z.B. Isopropylamino, Butylamino, Isobutylamino, sek.-Butylamino, tert.-Butylamino, Diisopropylamino, Dibutylamino, Diisobutylamino, Di-sek.-butylamino, Di-tert.-butylamino, Dibenzylamino, N-Benzyl-N-propylamino, N-Benzyl-N-butylamino, N-Benzyl-N-isobutylamino, N-Benzyl-N-sek.-butylamino, N-Benzyl-N-tert.-butylamino.

Besonders bevorzugte Reste R sind Cl und $H_2N$, ferner F, Methylamino, Ethylamino, Benzylamino, N-Benzyl-N-methylamino oder N-Benzyl-N-ethylamino.

Die Reste $R^1$ und $R^2$ sind bevorzugt H, Methyl, Ethyl oder Benzyl. $R^3$ ist bevorzugt Alkyl, insbesondere Methyl. $R^4$ und $R^5$ sind jeweils bevorzugt H oder $CH_3$, insbesondere H.

Gegenstand der Erfindung sind insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln Ia bis Ig ausgedrückt werden, die der Formel I entspre chen und worin die nicht näher bezeichneten Reste die bei der Formel I angegebene Bedeutung haben, worin jedoch

in Ia
$R^3$ Alkyl mit 1-4 C-Atomen bedeutet;
in Ib
$R^3$ $CH_3$ bedeutet;
in Ic
R F, Cl, $H_2N$, Methylamino, Ethylamino, Benzylamino, N-Benzyl-N-methylamino oder N-Benzyl-N-ethylamino,
$R^3$ und $R^5$ jeweils H oder $CH_3$ und
$R^4$ H bedeuten;
in Id
R F, Cl, $H_2N$, Methylamino, Ethylamino, Benzylamino, N-Benzyl-N-methylamino oder N-Benzyl-N-ethylamino,
$R^3$ $CH_3$,
$R^4$ H und
$R^5$ H oder $CH_3$ bedeuten;
in Id
R F, Cl, Br oder J bedeutet;
in Ie
R $R^1R^2N$ bedeutet;
in If
R F, Cl, $H_2N$, N-Benzyl-N-methylamino oder N-Benzyl-N-ethylamino,
$R^3$ $CH_3$,
$R^4$ H und
$R^5$ H oder $CH_3$ bedeuten;
in Ig
R F oder Cl,
$R^3$ $CH_3$ und
$R^4$ und $R^5$ jeweils H bedeuten.

Die Verbindungen der Formel I werden im übrigen nach an sich bekannten Methoden hergestellt, wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kan man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt. Andererseits ist es möglich, die Reaktion stufenweise durchzuführen, wobei man weitere Zwischenprodukte

isolieren kann.

Gegenstand der Erfindung ist ferner ein Verfahren zur Herstellung von Pyridazinonderivaten der Formel I sowie von deren Salzen, dadurch gekennzeichnet, daß man eine Ketosäure der Formel II

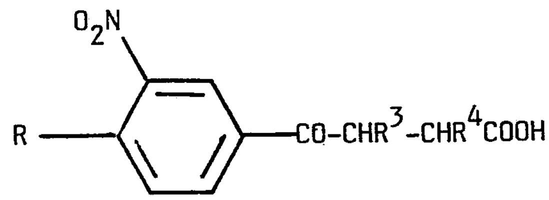

II

worin

R, $R^3$ und $R^4$ die angegebenen Bedeutungen haben

oder eines ihrer reaktionsfähigen Derivate mit einem Hydrazin der Formel $R^5$-NH-$NH_2$ (worin $R^5$ die angegebene Bedeutung hat) oder einem seiner reaktionsfähigen Derivate umsetzt

und/oder daß man in einer Verbindung der Formel I eine funktionelle Gruppe in eine andere funktionelle Gruppe umwandelt

und daß man gegebenenfalls eine Base der Formel I durch Behandeln mit einer Säure in eines ihrer Salze umwandelt.

Die Carbonsäuren der Formel II können nach an sich bekannten Methoden hergestellt werden, beispielsweise analog DE-OS 28 37 161.

Als reaktionsfähige Derivate der Carbonsäuren der Formel II eignen sich insbesondere die Ester, z.B. die Alkylester, worin die Alkylgruppe vorzugsweise 1-4 C-Atome besitzt, insbesondere die Methyl-und Ethylester, ferner die Nitrile, die Anhydride, die Säurehalogenide, z.B. Säurechloride oder Säurebromide, und die Amide. Weitere geeignete reaktionsfähige Derivate der Carbonsäuren der Formel II können während der Rekation in situ gebildet werden, ohne daß man sie isoliert. Dazu gehören beispielsweise die Hydrazone der Formel Ar-C(=$NNHR^5$)-$CHR^3$-$CHR^4$-COOH und die Hydrazide der Formel Ar-CO-$CHR^3$-$CHR^4$-CO-$NHNHR^5$ (worin Ar den 3-Nitro-4-R-phenylrest bedeutet).

Als reaktionsfähige Derivate des Hydrazins der Formel $R^5$-NH-$NH_2$ eignen sich z.B. die entsprechenden Hydrazinhydrate, Acethydrazide, Semicarbazide oder Carbazinsäureester.

Für die Umsetzung mit den Carbonsäuren der Formel II bzw. ihren reaktionsfähigen Derivaten verwendet man vorteilhaft 1-5 Äquivalente des Hydrazins bzw. reaktionsfähigen Hydrazinderivats, das gleichzeitig als Lösungsmittel dienen kann. Zweckmäßiger ist es jedoch, ein zusätzliches inertes Lösungsmittel zuzusetzen. Als inerte Lösungsmittel eignen sich vorzugsweise Alkohole wie Methanol, Ethanol, Isopropanol, n-Butanol, Isoamylalkohol, Glykole und deren Ether wie Ethylenglykol, Diethylenglykol, Ethylenglykolmonomethyl-oder -monoethylether (Methylglykol oder Ethylglykol), Carbonsäuren wie Ameisen-, Essig-oder Propionsäure, ferner Ether, insbesondere wasserlösliche Ether wie Tetrahydrofuran, Dioxan oder Ethylenglykoldimethylether (Diglyme); ferner Wasser sowie Gemische dieser Lösungsmittel untereinander, insbesondere Gemische mit Wasser, z.B. wässeriges Ethanol. Man kann auch eine Säure wie Schwefelsäure oder p-Toluolsulfonsäure als Katalysator zusetzen. Die Reaktionstemperaturen liegen zweckmäßig zwischen 0 und 200°, vorzugsweise zwischen 20 und 100°, die Reaktionszeiten zwischen etwa 1 und 48 Stunden.

Man kann ferner in an sich bekannter Weise in einer Verbindung der Formel I eine funktionelle Gruppe in eine andere funktionelle Gruppe umwandeln.

So kann man insbesondere ein Halogenatom (R = F, Cl, Br oder J) durch Umsetzung mit Ammoniak oder einem Amin der Formel $R^1R^2NH$ (z.B. Methyl-, Ethyl-, Benzyl-, N-Benzyl-N-methyl-, N-Benzyl-N-ethylamin) in eine Gruppe R = $R^1R^2N$ umwandeln. Diese Umsetzung wird zweckmäßig in einem der angegebenen inerten Lösungsmittel, vorzugs weise Methanol, Ethanol, Isopropanol oder n-Butanol mit oder ohne Zusatz von Wasser, bei Temperaturen zwischen etwa 0 und etwa 150, vorzugsweise zwischen 20 und 120°, und bei Drucken zwischen 1 und 100, vorzugsweise 1 und 5 bar, durchgeführt. Ein Überschuß des Amins kann auch als Lösungsmittel dienen.

Eine Base der Formel I kann mit einer Säure in das zugehörige Säureadditionssalz überführt werden. Für diese Umsetzung kommen insbesondere Säuren in Frage, die physiologisch unbedenkliche Salze

liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, aromatische oder heterocyclische ein-oder mehrbasige Carbon-, Sulfon-oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, Ascorbinsäure, Nicotinsäure, Isonicotinsäure, Methanoder Ethansulfonsäure, Ethandisulfonsäure, 2-Hydroxyethansulfonsäure, Benzolsulfonsäure, p-Toluolsulfonsäure, Naphthalin-mono-und disulfonsäuren, Laurylschwefelsäure. Salze mit physiologisch nicht unbedenklichen Säuren, z.B. Pikrate, können zur Aufreinigung der Verbindungen der Formel I verwendet werden.

Die freien Basen der Formel I können, falls erwünscht, aus ihren Salzen durch Behardlung mit starken Basen wie Natrium-oder Kaliumhydroxid, Natrium-oder Kaliumcarbonat in Freiheit gesetzt werden.

Verbindungen der Formel I können ein oder mehrere chirale Zentren enthalten. In diesem Fall liegen sie gewöhnlich in racemischer Form vor. Erhaltene Racemate können nach an sich bekannten Methoden mechanisch oder chemisch in ihre Enantiomeren getrennt werden. Vorzugsweise werden aus dem racemischen Gemisch durch Umsetzung mit einem optisch aktiven Trennmittel Diastereomere gebildet. Als Trennmittel eignen sich z.B. optisch aktive Säuren, wie die D-und L-Formen von Weinsäure, Diacetylweinsäure, Dibenzoylweinsäure, Mandelsäure, Äpfelsäure, Milchsäure oder die verschiedenen optisch aktiven Camphersulfonsäuren wie $\beta$-Camphersulfonsäure. Gut zur Trennung geeignet sind auch optisch aktive Sorptionsmittel.

Natürlich ist es auch möglich, optisch aktive Verbindungen der Formel I nach den oben beschriebenen Methoden zu erhalten, indem man Ausgangsstoffe verwendet, die bereits optisch aktiv sind.

Gegenstand der Erfindung ist ferner die Verwendung der Verbindungen der Formel I und ihrer physiologisch unbedenklichen Salze zur Herstellung pharmazeutischer Zubereitungen, insbesondere auf nicht-chemischem Wege. Hierbei können sie zusammen mit mindestens einem festen, flüssigen und/oder halbflüssigen Träger-oder Hilfsstoff und gegebenenfalls in Kombination mit einem oder mehreren weiteren Wirkstoffen in eine geeignete Dosierungsform gebracht werden.

Gegenstand der Erfindung sind ferner pharmazeutische Zubereitungen, enthaltend mindestens eine Verbindung der Formel I und/oder eines ihrer physiologisch unbedenklichen Salze.

Diese Zubereitungen können als Arzneimittel in der Human-oder Veterinärmedizin verwendet werden. Als Trägerstoffe kommen organische oder anorganische Substanzen in Frage, die sich für die enterale (z.B. orale), parenterale oder topische Applikation eignen und mit den neuen Verbindungen nicht reagieren, beispielsweise Wasser, pflanzliche Öle, Benzylalkohole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Dragees, Kapseln, Sirupe, Säfte oder Tropfen, zur rektalen Anwendung Suppositorien, zur parenteralen Anwendung Lösungen, vorzugsweise ölige oder wässerige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs-und/oder Netzmittel, Emulgatoren, Salze zur Beeinflussung des osmotischen Druckes, Puffersubstanzen, Farb-, Geschmacks-und/oder Aromastoffe enthalten. Sie können, falls erwünscht, auch einen oder mehrere weitere Wirkstoffe enthalten, z.B. ein oder mehrere Vitamine.

Gegenstand der Erfindung ist ferner die Verwendung der Verbindungen der Formel I bei der Bekämpfung von Krankheiten, insbesondere von Herzinsuffizienz, sowie ihre Verwendung bei der therapeutischen Behandlung des menschlichen oder tierischen Körpers.

Dabei werden die erfindungsgemäßen Substanzen in der Regel in Analogie zu bekannten positiv inotrop wirksamen Substanzen wie Amrinon verabreicht, vorzugsweise in Dosierungen zwischen etwa 1 und 100 mg, insbesondere zwischen 2 und 20 mg pro Dosierungseinheit. Die tägliche Dosierung liegt vorzugsweise zwischen etwa 0,02 und 2 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt. Im Vergleich zu den bisher zur Therapie der Herzinsuffizienz verwendeten Digitalis-Glykosiden zeichnen sich die Verbindungen der Formel I durch verbesserte therapeutische Breite und periphere Entlastung aus.

Vor-und nachstehend sind alle Temperaturen in Celsiusgraden angegeben.

Beispiel 1

Ein Gemisch von 6,8 l Essigsäure und 499 g Hydrazinhydrat wird mit 920 g 3-(4-Chlor-3-nitrobenzoyl)-buttersäure versetzt und bei 95-100° 2 Std. gerührt. Man gießt auf Eiswasser, filtriert ab und erhält 6-(4-Chlor-3-nitrophenyl)-5-methyl-2,3,4,5-tetrahydropyridazin-3-on, F. 186-188°.

Analog erhält man

6-(4-Chlor-3-nitrophenyl)-2,3,4,5-tetrahydropyridazin-3-on
6-(4-Fluor-3-nitrophenyl)-5-methyl-2,3,4,5-tetrahydropyridazin-3-on, F. 190-193°
6-(4-Brom-3-nitrophenyl)-5-methyl-2,3,4,5-tetrahydropyridazin-3-on
6-(4-Jod-3-nitrophenyl)-5-methyl-2,3,4,5-tetrahydropyridazin-3-on
6-(4-Chlor-3-nitrophenyl)-5-ethyl-2,3,4,5-tetrahydropyridazin-3-on.

Beispiel 2

Analog Beispiel 1 erhält man mit Methylhydrazin das 6-(4-Chlor-3-nitrophenyl)-2,5-dimethyl-2,3,4,5-tetrahydropyridazin-3-on, F. 91-92°.

Analog erhält man

6-(4-Chlor-3-nitrophenyl)-2-methyl-2,3,4,5-tetrahydropyridazin-3-on
6-(4-Fluor-3-nitrophenyl)-2,5-dimethyl-2,3,4,5-tetrahydropyridazin-3-on
6-(4-Brom-3-nitrophenyl)-2,5-dimethyl-2,3,4,5-tetrahydropyridazin-3-on
6-(4-Jod-3-nitrophenyl)-2,5-dimethyl-2,3,4,5-tetrahydropyridazin-3-on
6-(4-Chlor-3-nitrophenyl)-2-ethyl-5-methyl-2,3,4,5-tetrahydropyridazin-3-on.

Beispiel 3

Ein Gemisch von 5 g 6-(4-Chlor-3-nitrophenyl)-5-methyl-2,3,4,5-tetrahydropyridazin-3-on, 50 ml 25 %ig. wässeriger $NH_3$-Lösung und 50 ml Ethanol wird 16 Std. auf 100° erhitzt. Man gießt auf Eiswasser, extrahiert mit Dichlormethan, trennt ab, trocknet die organische Phase über Natriumsulfat, dampft ein und erhält 6-(4-Amino-3-nitrophenyl)-5-methyl-2,3,4,5-tetrahydropyridazin-3-on, F. 234-238°.

Analog erhält man mit Ammoniak oder den entsprechenden Aminen:

6-(4-Amino-3-nitrophenyl)-2,3,4,5-tetrahydropyridazin-3-on
6-(4-Methylamino-3-nitrophenyl)-5-methyl-2,3,4,5-tetrahydropyridazin-3-on
6-(4-Ethylamino-3-nitrophenyl)-5-methyl-2,3,4,5-tetrahydropyridazin-3-on
6-(4-Dimethylamino-3-nitrophenyl)-5-methyl-2,3,4,5-tetrahydropyridazin-3-on
6-(4-N-Benzyl-N-methylamino-3-nitrophenyl)-5-methyl-2,3,4,5-tetrahydropyridazin-3-on, F. 136-138°
6-(4-N-Benzyl-N-ethylamino-3-nitrophenyl)-5-methyl-2,3,4,5-tetrahydropyridazin-3-on, F. 138-140° ("B")
6-(4-Dibenzylamino-3-nitrophenyl)-5-methyl-2,3,4,5-tetrahydropyridazin-3-on, F. 161-163°.

Verwendungsbeispiel

Man löst 100 g 6-(4-Amino-3-nitrophenyl)-5-methyl-2,3,4,5-tetrahydropyridazin-3-on in 2 l Methanol und hydriert an 20 g 5 %iger Palladium-Kohle bei 20° und 1 bar bis zum Stillstand. Nach Filtration und Eindampfen erhält man 6-(3,4-Diaminophenyl)-5-methyl-2,3,4,5-tetrahydropyridazin-3-on, F. 195-196°.

Analog erhält man durch Hydrierung von 6-(4-N-Benzyl-N-methylamino-3-nitrophenyl)-5-methyl-2,3,4,5-tetrahydropyridazin-3-on [bzw. "B"] das 6-(3-Amino-4-methylaminophenyl)-[bzw. 6-(3-Amino-4-ethylaminophenyl)-]5-methyl-2,3,4,5-tetrahydropyridazin-3-on.

Die nachstehenden Beispiele betreffen pharmazeutische Zubereitungen, die Verbindungen der Formel I oder ihre Säureadditionssalze enthalten:

Beispiel A: Tabletten

Ein Gemisch von 1 kg "B", 4 kg Lactose, 1,2 kg Kartoffelstärke, 0,2 kg Talk und 0,1 kg Magnesiumstearat wird in üblicher Weise zu Tabletten gepreßt, derart, daß jede Tablette 10 mg Wirkstoff enthält.

Beispiel B: Dragees

Analog Beispiel A werden Tabletten gepreßt, die anschließend in üblicher Weise mit einem Überzug aus Saccharose, Kartoffelstärke, Talk, Tragant und Farbstoff überzogen werden.

Beispiel C: Kapseln

1 kg "B" wird in üblicher Weise in Hartgelatinekapseln gefüllt, so daß jede Kapsel 5 mg Wirkstoff enthält.

Beispiel D: Ampullen

Eine Lösung von 1 kg "B"-Hydrochlorid in 100 l zweifach destilliertem Wasser wird steril filtriert, in Ampullen abgefüllt, unter sterilen Bedingungen lyophilisiert und steril verschlossen. Jede Ampulle enthält 2 mg Wirkstoff.

Analog sind Tabletten, Dragees, Kapseln und Ampullen erhältlich, die einen oder mehrere der übrigen Wirkstoffe der Formel I und/oder ihre physiologisch unbedenklichen Säureadditionssalze enthalten.

**Ansprüche**

1. Pyridazinonderivate der Formel I

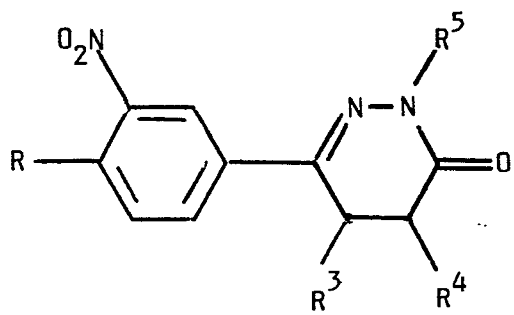

I

worin

R F, Cl, Br, J oder $R^1R^2N$,

$R^1$ und $R^2$ jeweils H, Alkyl oder Benzyl,

$R^3$, $R^4$ und $R^5$ jeweils H oder Alkyl bedeuten und

die Alkylgruppen jeweils 1-4 C-Atome enthalten, worin jedoch nur dann gleichzeitig R = Benzylamino, $R^3$ = $CH_3$ und $R^4$ = H sein können, wenn $R^5$ Alkyl bedeutet,

sowie deren Salze.

2.

a) 6-(4-Chlor-3-nitrophenyl)-5-methyl-2,3,4,5-tetrahydropyridazin-3-on,

b) 6-(4-Amino-3-nitrophenyl)-5-methyl-2,3,4,5-tetrahydropyridazin-3-on.

3. Verfahren zur Herstellung von Pyridazinonderivaten der Formel I sowie von deren Salzen, dadurch gekennzeichnet, daß man eine Ketosäure der Formel II

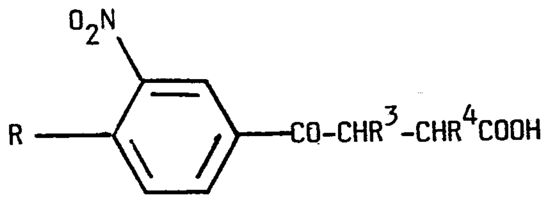

II

worin

R, $R^3$ und $R^4$ die angegebenen Bedeutungen haben

oder eines ihrer reaktionsfähigen Derivate mit einem Hydrazin der Formel $R^5$-NH-$NH_2$ (worin $R^5$ die angegebene Bedeutung hat) oder einem seiner reaktionsfähigen Derivate umsetzt

und/oder daß man in einer Verbindung der Formel I eine funktionelle Gruppe in eine andere funktionelle Gruppe umwandelt

und daß man gegebenenfalls eine Base der Formel I durch Behandeln mit einer Säure in eines ihrer Salze umwandelt.

4. Verfahren zur Herstellung pharmazeutischer Zubereitungen, dadurch gekennzeichnet, daß man eine Verbindung der Formel I und/oder eines ihrer physiologisch unbedenklichen Salze zusammen mit mindestens einem festen, flüssigen oder halbflüssigen Träger-oder Hilfsstoff und gegebenenfalls in Kombination mit einem oder mehreren weiteren Wirkstoff(en) in eine geeignete Dosierungsform bringt.

5. Pharmazeutische Zubereitung, gekennzeichnet durch einen Gehalt an mindestens einer Verbindung der Formel I und/oder einem ihrer physiologisch unbedenklichen Salze.

6. Verbindungen der Formel I zur Bekämpfung von Krankheiten des Herz-und Kreislaufs.

7. Verwendung von Verbindungen der Formel I zur Herstellung eines Arzneimittels.

8. Verwendung von Verbindungen der Formel I bei der Bekämpfung von Krankheiter des Herz-und Kreislaufs.